# EUROPEAN PATENT SPECIFICATION

(11) **EP 3 968 842 B1**
(45) Date of publication and mention of the grant of the patent: **01.07.2026**
(21) Application number: 20805563.2
(22) Date of filing: 12.05.2020
(51) Int. Cl.: A61B 3/16, A61B 3/00, A61B 3/10, A61B 5/00, A61F 9/00

(54) **SYSTEMS, DEVICES AND METHODS FOR OPTICAL INTERROGATION OF AN IMPLANTABLE INTRAOCULAR PRESSURE SENSOR**
SYSTEME, VORRICHTUNGEN UND VERFAHREN ZUR OPTISCHEN ABFRAGE EINES IMPLANTIERBAREN INTRAOKULAREN DRUCKSENSORS
SYSTÈMES, DISPOSITIFS ET PROCÉDÉS D'INTERROGATION OPTIQUE D'UN CAPTEUR DE PRESSION INTRAOCULAIRE IMPLANTABLE

(30) Priority: 13.05.2019 US 201962847127 P
(43) Date of publication of application: 23.03.2022
(73) Proprietor: Verily Health Inc., Dallas, Texas 75019 (US)
(72) Inventor: SINHA, Supriyo, South San Francisco, California 94080 (US); AZAR, Dimitri, South San Francisco, California 94080 (US); RUMYANTSEV, Oleg, South San Francisco, California 94080 (US)
(74) Representative: Mewburn Ellis LLP
(86) International application number: PCT/US2020/032510
(87) International publication number: WO 2020/232015

(56) References cited:
- US-A1- 2011 160 561
- US-A1- 2011 160 561
- US-A1- 2013 253 405
- US-A1- 2016 015 266
- US-A1- 2017 164 831
- US-A1- 2017 215 727
- US-A1- 2018 214 025
- LEE, J.O. ET AL.: "A microscale optical implant for continuous in vivo monitoring of intraocular pressure", MICROSYSTEMS & NANOENGINEERING, vol. 3, 18 December 2017 (2017-12-18), XP055762807

## Description

### TECHNICAL FIELD

The subject matter described herein relates to systems, devices, and methods for measuring intraocular pressure (IOP) in human eyes. These systems, devices, and methods have particular, but not exclusive, utility for treating eye diseases, including but not limited to glaucoma.

### BACKGROUND

Intraocular pressure (IOP) quantifies the pressure of fluid inside the eye. Many individuals suffer from disorders, such as glaucoma, that cause chronic heightened IOP. Over time, heightened IOP can cause damage to the optical nerve of the eye, leading to loss of vision. Presently, treatment of glaucoma mainly involves periodically administering pharmaceutical agents to the eye to decrease IOP. These drugs can be delivered, for example, by injection or eye drops. However, effective treatment of glaucoma requires adherence to dosage schedules and a knowledge of the patient's IOP. The more current or recent the measurement is, the more relevant it will be and hence the more effective the resulting treatment can be. The IOP for a given patient can vary significantly based on time of day, exercise, recency of medication use, and other factors. This means that any given measurement is subject to uncertainty, so it may take a plurality of measurements over time to provide confidence as to the health status of the patient. IOP measurements performed in a doctor's office typically only take place once or twice per year. These infrequent measurements are less able to account for variation in patient IOP. Annual or biannual measurements in a doctor's office may also grow stale or obsolete due to time lag since the previous measurement. Frequent measurements at home could allow for better treatment at lower cost.

IOP may be measured through tonometry, however commonly used tonometry techniques and devices have numerous drawbacks. Contact tonometry is performed in a medical setting and carries both an infection risk and a risk of injury. The procedure may also require numbing of the patient's eyes, resulting in both inconvenience and discomfort. Noncontact tonometry involves directing a puff or jet of air at the patient's eye and measuring the resulting deflection of the eye. However, this requires piston pumps with fast acceleration, which in turn requires a large solenoid, high coil force and high electrical power, and thus a relatively large device to accommodate a coil, piston, and power supply. Accordingly, such pumps can be relatively heavy and expensive. The cost, weight, size, and power consumption of non-contact tonometers make them impractical for home use. In addition, noncontact tonometry is often not as accurate as contact tonometry. Accordingly, long-felt needs exist for IOP measurement tools that address the forgoing and other concerns.

The information included in this Background section of the specification, including any references cited herein and any description or discussion thereof, is included for technical reference purposes only and is not to be regarded as subject matter by which the scope of the disclosure is to be bound.

US 2011/160561 Al discloses a system, device, and method for determination of intraocular pressure wherein multiple wavelengths are emitted sequentially.

US 2016/015266 A1 discloses systems and methods for sensing intraocular pressure wherein a reader unit scans an implant over a range of wavelengths.

### SUMMARY

Disclosed here are systems, devices, and methods for measuring intraocular pressure (IOP) in human eyes,

According to one embodiment, an intraocular pressure measurement system is presented as defined in claim 1.

According to another embodiment, a method for measuring an intraocular pressure of an eye using a pressure sensor having an optical cavity and implanted in the eye is presented as defined in claim 10.

This Summary is provided to introduce a selection of concepts in a simplified form that are further described below in the Detailed Description. This Summary is not intended to identify key features or essential features of the claimed subject matter, nor is it intended to limit the scope of the claimed subject matter. A more extensive presentation of features, details, utilities, and advantages of the IOP measurement system, as defined in the claims, is provided in the following written description of various embodiments of the disclosure and illustrated in the accompanying drawings.

### BRIEF DESCRIPTION OF THE DRAWINGS

Illustrative embodiments of the present disclosure will be described with reference to the accompanying drawings, of which:
**Figure 1** is a diagrammatic, cross-sectional representation of an example IOP measurement system in accordance with at least one embodiment of the present disclosure.
**Figure 2** is an exemplary spectral response of the light reflected from an implantable sensor of an IOP measurement system in accordance with at least one embodiment of the present disclosure.
**Figure 3** is a diagrammatic, cross-sectional view of an example IOP measurement system, including the eye, sensor, light source, and spectrometer according to at least one embodiment of the present disclosure.
**Figure 4** is a diagrammatic, cross-sectional view of an example IOP measurement system, including the eye, sensor, and incident light rays according to at least one embodiment of the present disclosure.
**Figure 5** is a diagrammatic, cross-sectional view of an example implantable pressure sensor according to at least one embodiment of the present disclosure.
**Figure 6** is a graphical representation of the spacing between adjacent spectral peaks, as a function of the thickness of the cavity, for different wavelengths of light, in accordance with at least one embodiment of the present disclosure.
**Figure** 7 is a graphical representation of the spacing between adjacent spectral peaks, as a function of the thickness of the cavity, for different deflections of the membrane, in accordance with at least one embodiment of the present disclosure.
**Figure 8** is a graphical representation of membrane deflection as a function of IOP for different membrane diameters, in accordance with at least one embodiment of the present disclosure.
**Figure 9** is a diagrammatic representation of a linear array of light beams reflecting from an implantable IOP sensor according to at least one embodiment of the present disclosure.
**Figure 10** illustrates various peak shifts for various membrane deflections at different membrane spacings in an example embodiment.
**Figure 11** is a method of measuring IOP in accordance with at least one embodiment of the present disclosure.

### DETAILED DESCRIPTION

The present disclosure describes systems, devices, and methods for optical interrogation of a pressure sensor implantable within the eye. Interferometry is employed on one or more reflections from the implantable sensor of light from a broadband (e.g., a source having a full-width-half-maximum bandwidth of 50 nm or more), spatially coherent light source (e.g., a superluminescent diode or SLD). The implantable pressure sensor may comprise a cavity (e.g., a low-finesse cavity) formed between a rigid substrate and a flexible membrane, whose deflection affects the depth of the cavity (i.e., the distance between the substrate and the membrane) and is indicative of IOP.

The light source may comprise an array (e.g., a linear array) of spatially coherent beams, forming small (e.g., close to the diffraction limit) spots of broadband light that may pass through the cornea, vitreous humor, and aqueous humor of the eye. In some embodiments, the implant is in the cornea or on top of the iris. In such embodiments, the light may still go through all of the structures but may not do so all of the time and may not in their intended use.

Power output of the light source may be selected such that spots that are incident on absorbing anatomical structures of the eye (e.g., the iris or retina) are not damaged by the incident energy. In an example, the system may generate near-diffraction-limited spots (e.g., with diameters less than 150% of the diffraction limited diameter), with power output of about 1-2 microwatts per spot, although other power outputs may be found to be advantageous. Spots that are incident on the implantable pressure sensor will reflect from the sensor and may be captured by a spectrometer located outside the eye (e.g., in a handheld device that also includes the light source). Readings from the spectrometer are indicative of IOP as described below.

In general, a reflection off a cavity will yield different measured distance between the substrate and membrane based on the viewing angle. For example, a head-on measurement (incident angle of 0 degrees, measured from the normal) will yield a first distance, whereas an incident angle of 45 degrees will yield a different distance due to the geometry of the beam and cavity. Generally, a measurement taken off-normal will mimic a shorter cavity. An incident angle close to 90 degrees will generally not return a reflection to the optical interrogation system. This presents a challenge to IOP measurement systems, as small movements of the eye or measuring device may change the incident angle and thus result in inaccurate readings.

However, the systems of the present disclosure may be relatively insensitive to lateral alignment, as the spacing between emitted beams from the light source may be less than the width or diameter of the implantable pressure sensor membrane.

Alternatively, or additionally, the systems may provide fiducial markers on the implantable sensor (e.g., an annulus surrounding the central cavity that has a depth that is independent of pressure), which can be detected by the spectrometer or by another sensor (e.g., a camera), such that the measured spectrum from the fiducials can be used to calculate the incidence angle. Fiducial markers provided immunity against angular misalignment. In summary, an array of spots or beams provides immunity against lateral misalignment, and one or more fiducial markers provide immunity against angular misalignment.

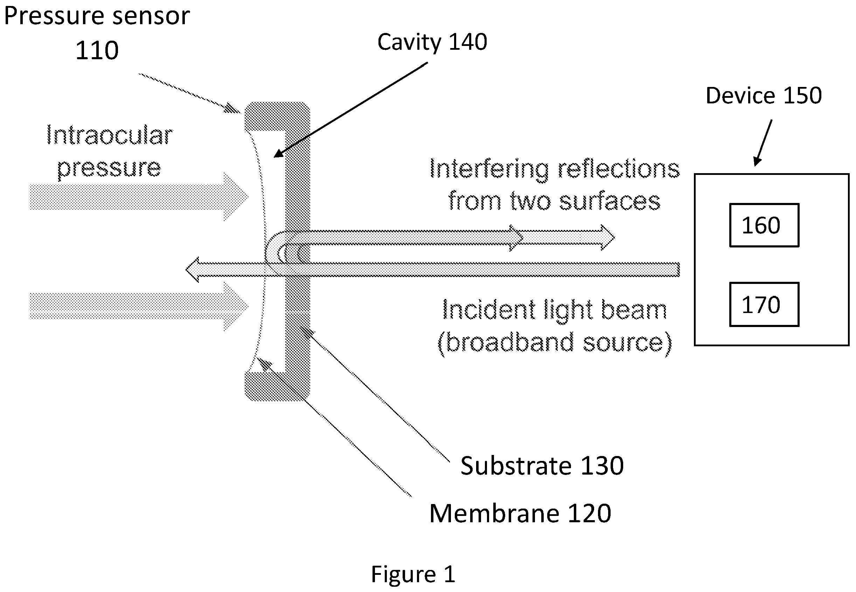

**Figure 1** is a diagrammatic, cross-sectional representation of an example IOP measurement system in accordance with at least one embodiment of the present disclosure. In this embodiment, the system employs a pressure sensor 110 implanted in an eye, and an external device 150 that includes a light source 160 and a spectrometer 170. The sensor 110 includes a diaphragm or membrane 120 and substrate 130 that together form an optical cavity 140. For example, as shown in Figure 1, a portion of a surface of a substrate 130 and a portion of a surface of membrane 120 may define an optical cavity 140. In an embodiment, a cross-section of the cavity 140 in a plane perpendicular to Figure 1 is circular.

The IOP measurement system may employ low coherence interferometry to obtain accurate readings of the intraocular pressure. Light with a broad spectrum may be incident on a cavity 140, such that only the primary reflections from two surfaces need to be considered: the surface of the substrate 130 that is closest to the light source, and the surface of the membrane 120 that is closest to the light source. The membrane 120 bends predictably and reversibly in proportion to increasing pressure (e.g., intraocular pressure), which in turn reduces the distance between the centers (or other corresponding points) of the two surfaces 120 and 130. For example, the distance between a point on the surface of membrane 120 and a point on a surface of the substrate 130 decreases (increases) as pressure increases (decreases). The membrane 120 and the substrate 130 differ in stiffness, such that for a range of expected IOP, the substrate exhibits little-to-no deflection, whereas the membrane exhibits measurable deflection. In various embodiments, the stiffness of the substrate 120 exceeds the stiffness of the membrane by a factor of 100 or 1000 or more. In operation, the membrane 120 exhibits flexibility with respect to IOP, whereas the substrate exhibits rigidity. The embodiment in Figure 1 illustrates light incident on the substrate 130 first, with light that is not reflected transmitted next to the membrane 120. In other embodiments, the light is incident on the membrane 120 first, with light that is not reflected transmitted next to the substrate 130. In such an embodiment, the sensor may be oriented so that it is rotated up to 180 degrees from the orientation shown in Figure 1.

Optical pressure sensors are known that employ a fiber optic coupling, meaning the light source and spectrometer are both attached to the pressure sensitive diaphragm. This is not practical for measurement of intraocular pressure, since the size of the source and a spectrometer with sufficient spectral resolution would be too large for a practical surgical implant. The present disclosure describes a system to interrogate a pressure sensor, such as sensor 110, through free space. Thus, the light source and spectrometer are located outside the eye, whereas the pressure sensor is implantable and located within the eye. This significantly relaxes size and power constraints of the system.

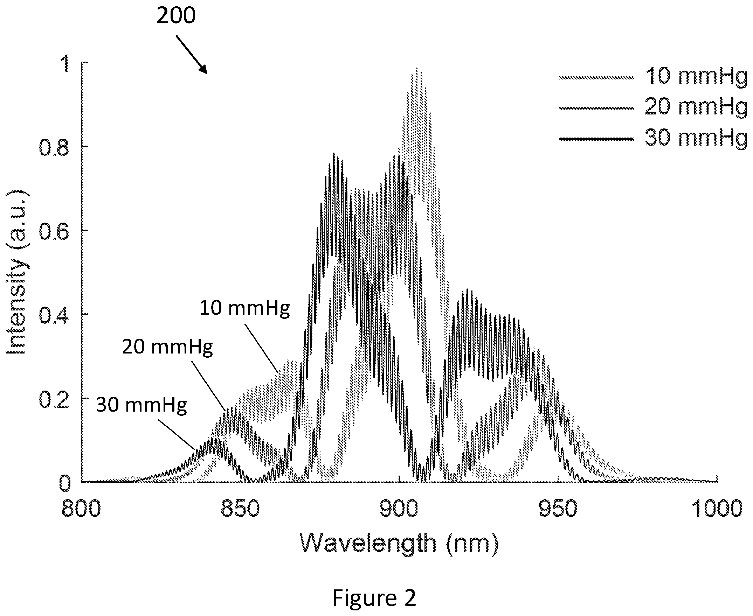

**Figure 2** is an exemplary spectral response 200 of the light reflected from an implantable sensor of an IOP measurement system in accordance with at least one embodiment of the present disclosure. The graph depicts an interference spectrum from an exemplary low-finesse Fabry Perot interferometer, for different values of IOP. As can be seen from the graph, for a given light source, a given cavity diameter and depth, and a given membrane thickness or flexibility, the locations of the peaks detected by the spectrometer can be related predictably to the intraocular pressure by a mathematical equation or lookup table.

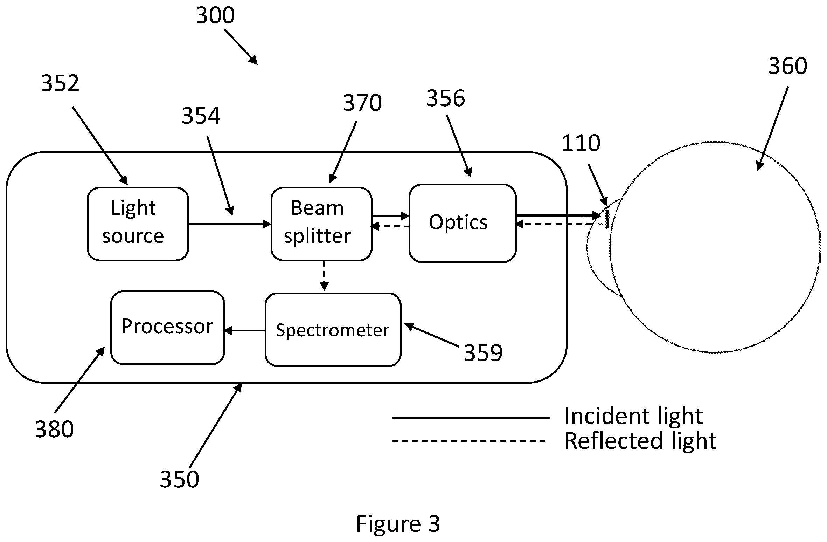

**Figure 3** is a diagrammatic, cross-sectional view of an example IOP measurement system 300, including sensor 110 implanted in the eye 360, and an external device 350 that includes a light source 352 emitting a pattern of beams 354 which passes through a beam splitter 370 and various other optical components 356 (such as lenses, collimators, etc.) and then strikes an implanted sensor 110 within the eye 360. Light reflected from the implanted sensor 110 passes back through optics 356 into a spectrometer 359 according to at least one embodiment of the present disclosure. The figure illustrates the sensor 110 in the eye with the light source and spectrometer 359 located within a single device 350 (e.g., a handheld wand) that is held close to, but outside of, the eye 360. In an embodiment, the sensor 110 is affixed to the edge of the iris, or to the inner surface of the cornea. The spectrometer 359 is coupled to a processor 380. The processor 380 is configured to receive an output, such as a spectrum measurement, and estimate the IOP. The processor 380 may be any type of known processing device, such as a programmed general-purpose processor, a digital signal processor, an application specific integrated circuit, or a field-programmable gate array.

In an embodiment, the external device 350 also includes a memory (not shown) that stores instructions for the processor 380. The memory may also store IOP measurement values. In an embodiment, the external device 350 also includes a communication device (not shown) that is capable of wireless communication using any known technique for one-way or two-way wireless communication, such as Bluetooth, Wi-Fi, or cellular communication. The communication device may be used to download software to the device 350 and/or may be used to communicate measured IOP values to another device, such as a smartphone. In an embodiment, the external device 350 also includes a display to display measured IOP values. The display may be any known display, such as light-emitting diode (including organic LED), or liquid crystal display (LCD). Thus, computed IOP values may be displayed on external device 350 and/or communicated to another device (e.g., a smartphone) for display.

One of the challenges for this type of optical sensing is to ensure that the measurement is not substantially affected by relative motion (translational or rotational) between the sensor and the light source/spectrometer. Thus, the system may need to know when it is properly interrogating the sensor as well as minimize the chances that motion would affect the result. To ensure the sensor is sufficiently interrogated, in some embodiments, the IOP measurement system projects a plurality of spots onto the eye in the plane that the sensor is believed to be located. A "spot" refers to a cross-section of a light beam. Examples of the plurality of spots include a line, a linear array of spots, a grid, or a two-dimensional (2D) array of spots. The plurality of spots may extend beyond the "active" area of the sensor so that fiducials could also be located that would have a characteristic signature on the spectrometer. In an example, the reflected light from the sensor and fiducial markers from a projected line would be dispersed across a two-dimensional sensor. These fiducials could also be used, for example, to calibrate tilt error for example. Alternately, the fact that the membrane would be flexing, bowing, or deforming in a spherical type shape allows the system to use that shape as a locating feature even in the absence of fiducials. In some embodiments, the system includes sufficient spatial resolution to discriminate the size and shape of the feature, and to discern the displacement of the membrane even at the lowest levels of IOP that are of clinical concern. For example, the displacement is measured relative to a reference displacement and associated pressure (e.g., zero pressure).

One method to bring the light source and spectrometer to the patient would be to have the system as a handheld device (e.g., a wand), although this approach may introduce motion artifacts and other inaccuracies under some circumstances. Alternatively, the light source and spectrometer may also be mounted in goggles or spectacles that retain a relatively fixed position with respect to the eye, thus significantly reducing any motion artifacts due to movement of the head.

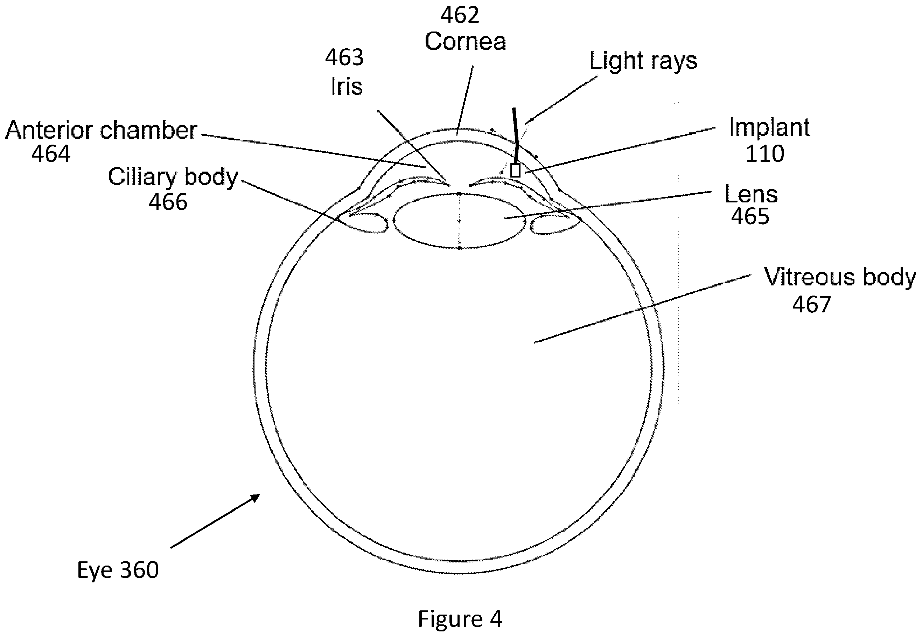

**Figure 4** is a diagrammatic, cross-sectional view of a sensor 110 implanted in the eye 360 according to at least one embodiment. Also visible are incident light rays entering the eye 360 (e.g., denoted as a line that bends upon entering the eye 360). The eye 360 comprises a cornea 462, iris 463, anterior chamber 464, lens 465, ciliary body 466, and vitreous body 467. Figure 4 illustrates an example placement of a pressure sensor 110 relative to the indicated parts of the eye.

In some embodiments, the placement and securing of a pressure sensor 110 within an eye 360 is selected according to various criteria. First, it may be desirable to ensure that the sensor 110 is securely placed and will not move within the eye 360 over time. Second, since it is desirable for the sensor to not obscure vision, the sensor may be placed in the periphery of the eyeball. If the sensor were located behind the iris 463 in a non-obscuring position, then it might be desirable for the pupils of the iris 463 to be fully dilated in order for the implantable pressure sensor 110 to be visible to the light source 352 and spectrometer 358. This may be inconvenient for patients and may be problematic for elderly patients in whom dilation response tends to be low.

Thus, in some embodiments it may be preferable to place the pressure sensor in front of the iris near the angle of the eye, oriented in such a way that light enters the sensor implant 110 at an angle well separated from any angles of total internal reflection, i.e., the angles at which light rays entering the eye or the implantable pressure sensor will be internally reflected and thus not escape back outside the eyeball where they can be read by the spectrometer. Figure 4 shows one possible placement of the implantable pressure sensor 110. In some embodiments, placement of the sensor is desirable in regions where iris contractions do not interfere with the functioning of the sensor.

In some embodiments, the system may scan a line across the eye 360 to find the proper location of the sensor 110. An alternate embodiment may have a point scanned across the eye to further reduce the background. In other embodiments, the implantable pressure sensor 110 may be placed within the cornea 462. Although a typical thickness of the cornea may be only ~0.5 mm at the center of the eye, the thickness increases to nearly 0.9 mm closer to the scleral boundary. Thus, it may be reasonable to place an implantable device 110 that is up to 0.3 mm thick and 1 mm in diameter into the cornea 462. Such a surgery may be less invasive and may allow for easier optical access to the implant. Still other embodiments may integrate both optical and electrical readout of the IOP. In this case, the flexible membrane is available for optical readout, this membrane could also be used in conjunction with a strain gauge or capacitive pressure readout.

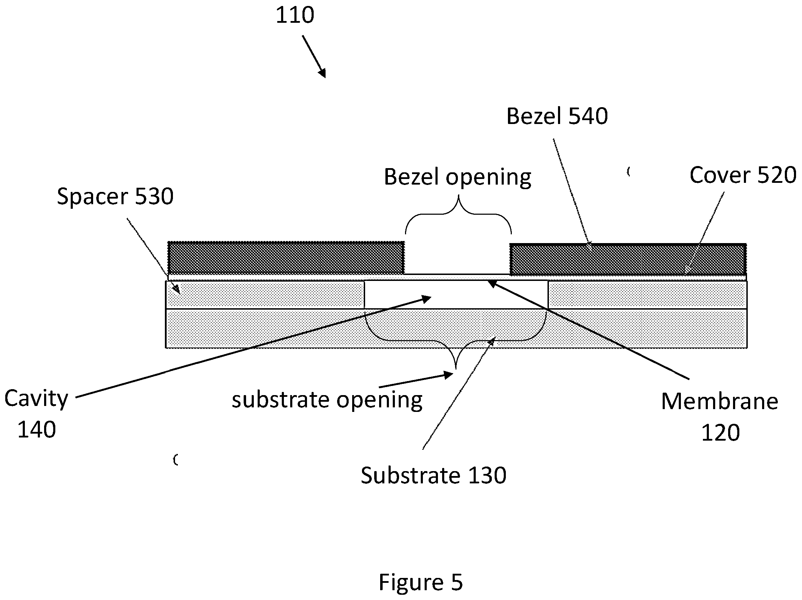

**Figure 5** is a diagrammatic, cross-sectional view of an example implantable pressure sensor 110 according to at least one embodiment. In the example shown in Figure 5, the implantable pressure sensor 110 comprises a substrate 130, a spacer or spacer layer 530 affixed on the substrate 130, a cover 520 affixed on top of the spacer, and then a bezel 540 disposed on the cover 520 and forming a top layer. The substrate 130 may be made from a transparent or opaque material in a variety of different thicknesses. In an example the substrate 130 is a quartz wafer with a thickness of between about 0.15 mm and 0.35 mm, while the spacer 530 is wafer bonded quartz with a thickness of between about 3 µm and 100 µm, the cover 520 is a layer of transparent, at least partially-reflective material (e.g., quartz) thin enough to serve as a flexible membrane 120, and the bezel 540 is silicon or quartz with a thickness of about 0.2 mm, although other thicknesses may be used.

In this example, the layers are circular, and the spacer 530 includes an opening (such as a circular opening) such that a cavity 140 is formed between the substrate 130, the spacer 530, and the cover 520. The bezel 540 also has an opening (such as a circular opening), such that a portion of the cover is exposed, forming a membrane that is able to flex in response to the pressure from fluid in the eye (e.g., intraocular pressure). In an embodiment, the opening in the bezel 540 is smaller in area than the opening on the spacer 530, and the opening in the bezel 540 is sufficiently aligned with the opening in the spacer 530 that a cross-sectional area of the bezel opening 540 completely overlaps a cross-sectional area of the spacer 530 opening.

To ensure long-term stability of the device, it is desirable to ensure that any gas that is in the cavity 140 does not diffuse out to the environment. Good practices of hermetic sealing should significantly reduce leakage. In addition, instead of air (comprising mostly small nitrogen and oxygen molecules), an insert gas may be used, such as SF6 or C3F8, which comprise larger gas molecules. These larger molecules would diffuse out much more slowly. Molecular weight or size is less relevant to the lifetime of the sensor if the cavity 140 is truly hermetically sealed, and more relevant to the lifetime of the sensor if the cavity 140 is less well sealed.

One of the challenges of this approach is to obtain a good signal-to-noise ratio (SNR) of the optical signal. Background reflections within the eye could reduce the SNR in some embodiments. The present disclosure helps to mitigate this issue with several techniques. These techniques may be employed individually or in combination. First, a low coherence source is employed in some embodiments, so that reflections that are more than a few hundred microns away from each other are unlikely to produce coherent artifacts that will influence the fringe pattern in the received spectrum. Second, an array of beams may be employed that are focused very tightly, and, as a result, the beam spread may reduce fringe visibility in regions that are not within the Rayleigh range of the focus. Also, the refractive index difference between the substrate and the surrounding ocular environment may be designed to be much smaller than the index difference between the substrate and the gas in the well. Likewise, the refractive index difference between the membrane and the surrounding ocular environment may be designed to be much smaller than the index difference between the substrate and the gas in the well. This reduces the magnitude of the reflection at the undesired interfaces.

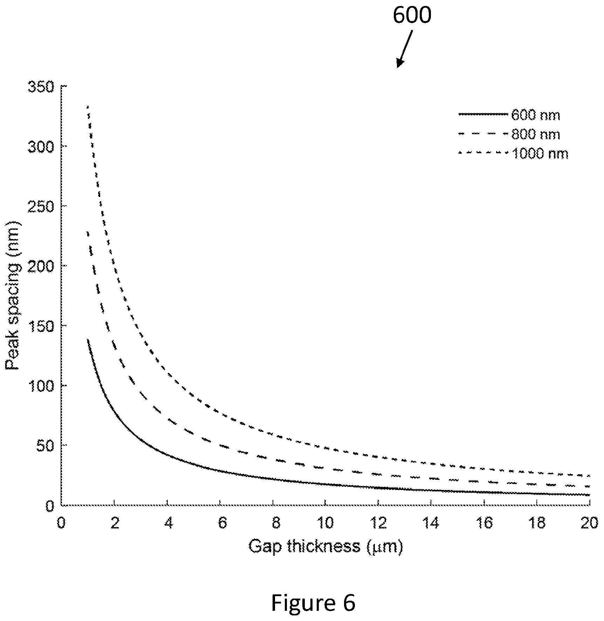

**Figure 6** is a graphical representation 600 of the spacing between adjacent spectral peaks, as a function of the thickness of the cavity, for different wavelengths of light, in accordance with at least one embodiment. This graph has been assembled from simulations of the system's performance to determine the operating space that is needed for a low cost implantable sensor, light source, and spectrometer combination.

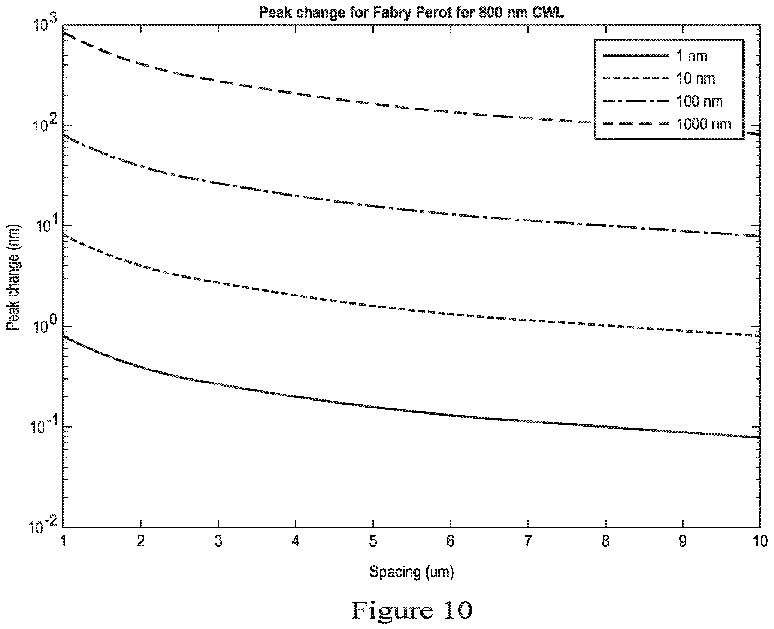

The graph shows the spacing between adjacent peaks at the interferogram measured at the spectrometer, as a function of cavity depth (identical to the spacer thickness or "spacing") and the center wavelength of the light source. The different curves each represent different center wavelengths. This result suggests that if an inexpensive light source is desired that has a bandwidth of about 30 nm, such that two distinct spectral peaks can be captured, a cavity depth of a few microns is sufficient, particularly if longer center wavelengths (e.g., near-infrared light with a center wavelength of 900 nm) are employed. In some embodiments, only one spectral peak is needed to deduce the membrane deflection and thus the IOP. In these cases, even thinner spacers may be used than are shown in this figure. **Figure 10** illustrates how the peak shifts for different deflections at different membrane spacings in an example embodiment.

The entire spectrum may provide information on the membrane deflection. This includes both peak separation (useful for broadband sources) or peak location (useful for more narrowband sources). In some embodiments, both could be used together. The choice could be dependent on the dynamic range of the deflection expected. If the source is narrow such that only a single peak is captured, then as the deflection increases, the adjacent peak would eventually move in to replace the peak in the non-deflected case. It could be difficult to distinguish between the two cases if you took snapshots of the two, but the two cases can be distinguished since the width of the peak is also slightly modified.

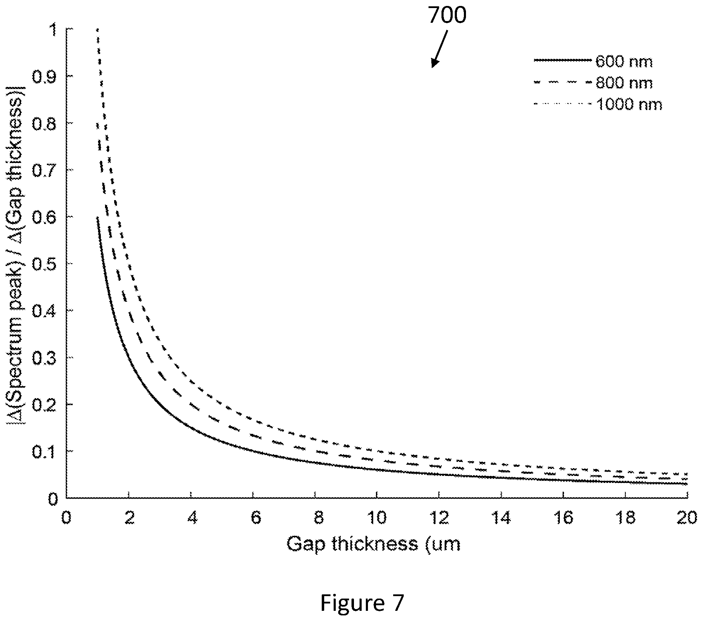

**Figure 7** is a graphical representation 700 of the spacing between adjacent spectral peaks, as a function of the thickness of the cavity, for different deflections of the membrane, in accordance with at least one embodiment. This graph shows how much the peak moves as a function of the spacing (i.e., spacer thickness or cavity depth). This graph demonstrates that for IOP-driven membrane displacements of about 10 nm, an implantable pressure sensor with a spacing of less than 10 µm is necessary to achieve a spectral peak spacing greater than about 1 nm. To achieve spectral peak spacing of 100 nm or greater, it may be necessary to have a spacing or cavity depth of less than 10 microns and a membrane deflection of 1000 nm or 1 um. This combination is not necessarily desirable, for reasons that will be discussed below.

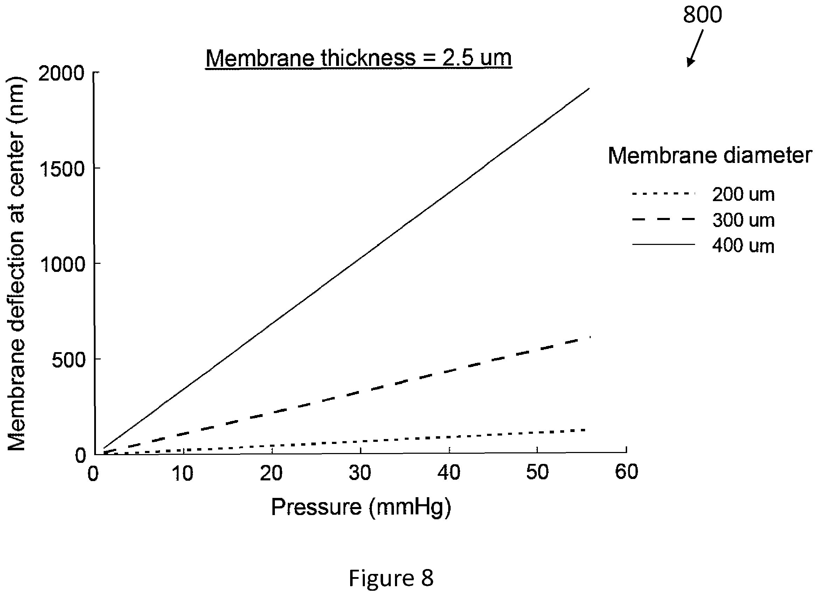

**Figure 8** is a graphical representation 800 of membrane deflection as a function of IOP for different membrane diameters, in accordance with at least one embodiment. The graph shows the displacement that would be expected for an SiO₂ (quartz) membrane at different IOP values for a given membrane thickness at different diameters. As can be seen in the graph, a 10 nm deflection is readily obtainable for membrane diameters of 0.2 mm or greater, whereas a deflection of 500 nm is only attainable for membrane diameters of 0.3 mm or greater, and only attainable at IOP values of 0-15 mmHg for membrane diameters of 0.4 mmHg or greater, which would require a device size that is not necessarily desirable for implantation in a living human eye. For embodiments where a transfer function of 1 nm of deflection per 1 mmHg of intraocular pressure, a membrane diameter of 0.2 mm is sufficient for a 2.5 µm thick membrane, and membrane diameters of slightly less than 0.2 mm (e.g., ~0.19 mm) may work as well. Similarly, for a maximum membrane deflection of ~500 nm at the center and sensitivity of 10 nm/mmHg, a diameter of ~0.3 mm would be sufficient.

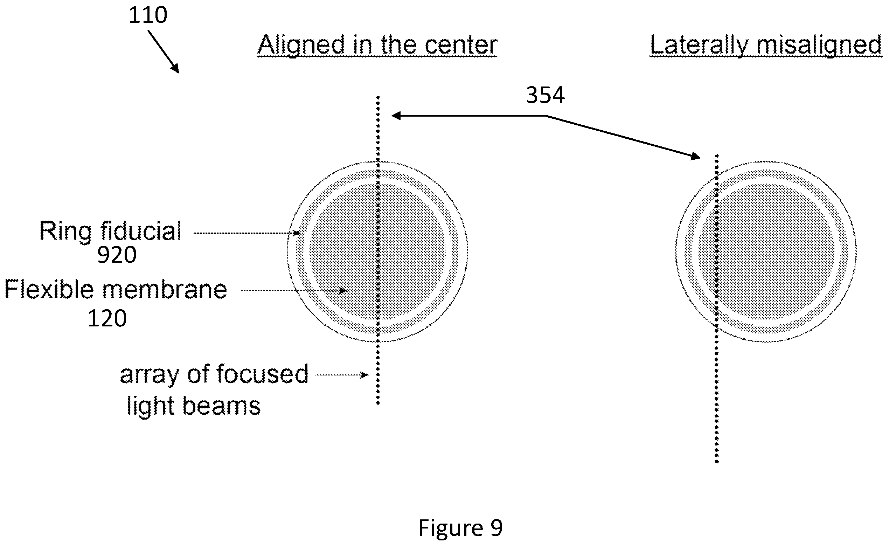

**Figure 9** is a top view of an implantable intraocular pressure sensor 110 illuminated by a linear array of light beams 354. Each of the dots in the indicated array of light beams represents a reflection of one of the beams. As shown, the sensor 110 includes membrane 120 and one or more fiducial markers 920. The light beams 354 reflect from an implantable IOP sensor 110 according to at least one embodiment of the present disclosure. In this example, the sensor is illuminated with a linear, or 1D, array of focused light beams 354. Even if the line is misaligned with respect to the center of the membrane 120, the position of spots hitting the membrane 120 can be determined from the number of spots with modulated spectrum and/or from the apparent distance between fiducial markers 920 along the line of the linear array (and also alternatively or additionally from spots hitting above the center of the membrane and from those below the center of the membrane). Fiducial markers 920 may, for example be ring fiducials located on the bezel.

In order to effectively record reflectance spectrograms (such as shown in Figure 2) a broadband light source of about 100 nm bandwidth may be employed. For example, a superluminescent diode (SLD) may be employed as the light source. This has several advantages over conventional broad-band sources (e.g. tungsten-halogen bulbs): high spatial coherence (e.g. ability to focus light into a tight diffraction-limited focal spot); high efficiency and low power consumption; and sufficient temporal coherence in order to observe interference of reflections from surfaces offset by up to about 100 µm.

Such a light source also offers the ability to pulse the illumination light, which allows illumination of the sensor only for a fraction of each spectrogram image capture time, for example for 1 microsecond during 1 millisecond spectrometer camera chip exposure. In such a manner, the system can capture a 'snapshot' of reflected light interference, not corrupted by motion artifacts. This also relaxes requirements for the camera chip acquisition rates (which may permit corresponding reductions in cost and complexity of the system).

There are several advantages to this proposed solution. First, the implanted surgical device is very simple. It has no electronics (passive or active). Thus, the implant can be made extremely compact with a high degree or reliability. Second, the complexity of the readout system is placed in the handheld/goggle device. This allows for repairs/upgrades to be done more easily. Furthermore, the system could have tiered readout systems where moderate accuracy readouts can be done at home (e.g., +/-2 mmHg) and high accuracy readouts (that might require more expensive electronics and/or a more time-consuming measurement) could be done in a doctor's office. Third, there is no need to communicate with the device or power the device using external wireless or RF power.

One of the challenges for optical readout is to ensure that the measurement is not impacted by relative motion (translational or rotational) between the sensor and the light source/spectrometer. This means that the system may need to detect when it is properly interrogating the sensor as well as minimize the chances that motion would affect the result. For example, when interrogating the membrane with a single focused light beam any deviation of focus position from the center (due to handheld misalignment) causes measurement error because the membrane deflection is not uniform across its area. In the context of a handheld measurement, this limitation becomes prohibitive.

To overcome the above limitation, the IOP measurement system of the present disclosure projects an array (such as a linear or 1D array) of spots onto the sensor implanted in the eye. The array of spots extends beyond the "active" area of the sensor (membrane) such that when it makes a cross-section of the membrane, a processor within the system can determine the length/position of the line and correctly convert the read-out signal into pressure reading (as shown in Figure 9). Any increase in the length of the array of spots beyond the diameter of the membrane provides additional dimensional tolerance in that dimension (parallel to the array of spots).

To determine the length of the line that hits the active area, the system can employ fiducials having a characteristic signature on the spectrometer and located on the perimeter of the sensor. The reflected light from the sensor and fiducials from the projected line may be spectrally dispersed across a 2D sensor. This approach allows the system to estimate the positions of the spots with modulated reflectance spectrum and consequently properly estimate the intraocular pressure causing membrane deflection. The linear array of spots can be measured from almost anywhere along the diameter of the membrane in a direction perpendicular to the array of spots, thereby increasing the dimensional tolerance in that dimension. Fiducials could also be used to compensate for the implant tilt. Alternatively, the system can use the number of spots with modulated spectrum to determine their positions on the membrane without referring to fiducials and use the asymmetry in their spectral profiles as before. Overall, interrogation of an implanted sensor with an array of spots (as opposed to a single spot) allows for a much greater volume (e.g., based on an increase in lateral area) where the handheld may be positioned to take the intraocular pressure measurement.

An alternate embodiment could have the system scan the line across the eye to find the proper location of the sensor. An alternate embodiment could have a point scanned across the eye to further reduce the background.

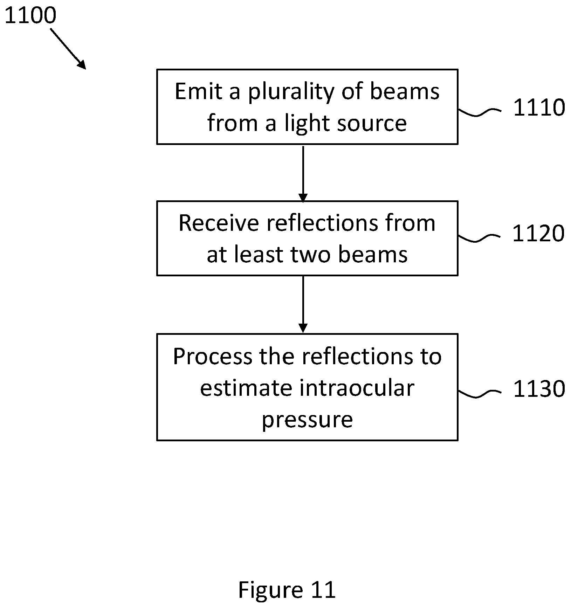

**Figure 11** illustrates a method 1100 of measuring IOP according to an embodiment. The method 1100 utilizes a system for measuring IOP, such as the example systems disclosed herein in **Figures 1** and **3****.** The system employs an implantable sensor and a device external to the eye. The device includes a light source emitting an array of beams. According to the method, in step 1110, a plurality of light beams is emitted from a light source, such as an external measurement device. In an embodiment, at least two of the light beams impinge on an implanted sensor, such as the sensors described herein, and are reflected. In step 1120, reflections are received from the at least two light beams. The reflections may be received by the external device that includes the light source. In step 1130, the reflections are processed to estimate the intraocular pressure in the eye.

Accordingly, it can be seen that an IOP measurement system fills a long-standing need in the art, by providing an implantable pressure sensor which can be read without contact to the eye, via a light source and spectrometer that are relatively insensitive to alignment or positioning errors and will yield consistent readings across a wide range of alignments and positions.

Generally, any creation, storage, processing, and/or exchange of user data associated with the method, apparatus, and/or system disclosed herein is configured to comply with a variety of privacy settings and security protocols and prevailing data regulations, consistent with treating confidentiality and integrity of user data as an important matter. For example, the apparatus and/or the system may include a module that implements information security controls to comply with a number of standards and/or other agreements. In some embodiments, the module receives a privacy setting selection from the user and implements controls to comply with the selected privacy setting. In other embodiments, the module identifies data that is considered sensitive, encrypts data according to any appropriate and well-known method in the art, replaces sensitive data with codes to pseudonymize the data, and otherwise ensures compliance with selected privacy settings and data security requirements and regulations.

A number of variations are possible on the examples and embodiments described above. The above specification, examples and data provide a complete description of the structure and use of exemplary embodiments of the IOP measurement system as defined in the claims. Although various embodiments of the claimed subject matter have been described above with a certain degree of particularity, or with reference to one or more individual embodiments, those skilled in the art could make numerous alterations to the disclosed embodiments without departing from the scope of the claimed subject matter.

## Claims

1. An intraocular pressure measurement system (300), comprising:
a pressure sensor (110) implantable in an eye (360), wherein the pressure sensor defines an optical cavity (140) with a depth that varies based on an intraocular pressure of the eye; and
an external device (150, 350) comprising:
a light source (352) configured to emit a plurality of beams (354) such that at least two non-overlapping beams of the plurality of beams strike and reflect from the pressure sensor;
a spectrometer (359) configured to receive reflected light coming from the at least two non-overlapping beams of the plurality of beams and produce an output based on the reflected light coming from the at least two non-overlapping beams of the plurality of beams, wherein the output varies based on the depth of the optical cavity; and
a processor (380) configured to receive the output and, based on the output, estimate the intraocular pressure of the eye.

2. The system of claim 1, wherein the pressure sensor comprises:
a substrate (130); and
a membrane (120) opposing the substrate, wherein the depth is a distance between the substrate and the membrane.

3. The system of claim 2, wherein the pressure sensor further comprises:
a spacer layer (530) located between the membrane and the substrate, wherein the spacer layer, the substrate and the membrane are bonded to define the optical cavity; and
a bezel (540) affixed to the membrane and comprising an opening for the at least two non-overlapping beams of the plurality of beams to impinge on the optical cavity.

4. The system of claim 1, wherein the pressure sensor comprises:
a substrate (130) forming one portion of the optical cavity;
a spacer (530) comprising a first central opening forming a second portion of the optical cavity;
a membrane (120) affixed to the spacer and forming a third portion of the optical cavity; and
a bezel (540) comprising a second central opening in alignment with the first central opening.

5. The system of claim 4, wherein the first central opening is circular and has a first diameter of between 0.19 millimeters (mm) and 0.35 mm, and the second central opening is circular and has a second diameter of between 0.19 mm and 0.35 mm that is equal to or less than the first diameter.

6. The system of claim 3, wherein the bezel comprises fiducial markers (920).

7. The system of claim 1, wherein the optical cavity has a circular cross section.

8. The system of claim 1, wherein the processor is configured to correct for angular misalignment between the spectrometer and the pressure sensor based on the output.

9. The system of claim 1, wherein the processor is further configured to correct for lateral displacement error between the spectrometer and the pressure sensor based on the output.

10. A method (1100) for measuring an intraocular pressure of an eye (360) using a pressure sensor (110) having an optical cavity (140) and implanted in the eye, the method comprising:
emitting light into the eye from a light source (352) configured to emit a plurality of beams (354) configured such that at least two non-overlapping beams strike and reflect from the pressure sensor;
receiving light reflected from the at least two non-overlapping beams into a spectrometer (359) to produce a spectrum, wherein the spectrum varies based on a depth of theoptical cavity of the pressure sensor; and
communicating an output based on the spectrum to a processor (380) configured to receive the output and, based on the output, estimate the intraocular pressure of the eye.

11. The method of claim 10, wherein the pressure sensor comprises:
a substrate (130);
a spacer layer (530) affixed to the substrate and comprising a first central opening;
a cover (520) comprising a semi-reflective material, wherein the cover is affixed to the spacer layer, and wherein a portion of the cover forms a flexible membrane (120); and
a bezel (540) affixed to the cover and comprising a second central opening, wherein the second central opening forms a perimeter of the flexible membrane.

12. The method of claim 10
wherein emitting the plurality of beams resides in emitting spatially coherent light in an array of beams, wherein the beams are sized and spaced such that at least two adjacent beams are separated by a distance less than a cross-sectional dimension of the pressure sensor.

13. The method of claim 12, wherein the light source has a bandwidth of about 30 nm, and the optical cavity of the pressure sensor has a cavity depth of a few microns.

14. The method of claim 12 or claim 13, wherein the pressure sensor includes fiducial markers (920), and wherein the output is based on reflections of one or more of the beams from the fiducial markers.

15. The method of claim 12 or claim 13, wherein the processor is further configured to correct for angular misalignment between the spectrometer and the pressure sensor.

## Patentansprüche

1. Augeninnendruck-Messsystem (300), umfassend:
einen Drucksensor (110), der in einem Auge (360) implantierbar ist, wobei der Drucksensor einen optischen Hohlraum (140) mit einer Tiefe definiert, die basierend auf einem Augeninnendruck des Auges variiert; und
eine externe Vorrichtung (150, 350), die Folgendes umfasst:
eine Lichtquelle (352), die ausgelegt ist, um eine Vielzahl von Strahlen (354) auszusenden, sodass zumindest zwei nichtüberlagernde Strahlen aus der Vielzahl von Strahlen auf den Drucksensor auftreffen und von diesem reflektiert werden;
ein Spektrometer (359), das ausgelegt ist, um das reflektierte Licht, das von den zumindest zwei nichtüberlagernden Strahlen aus der Vielzahl von Strahlen stammt, zu empfangen und um eine Ausgabe basierend auf dem reflektierten Licht, das von den zumindest zwei nichtüberlagernden Strahlen aus der Vielzahl von Strahlen stammt, zu erzeugen, wobei die Ausgabe basierend auf der Tiefe des optischen Hohlraums variiert; und
einen Prozessor (380), der ausgelegt ist, um die Ausgabe zu empfangen und den Augeninnendruck des Auges basierend auf der Ausgabe zu schätzen.

2. System nach Anspruch 1, wobei der Drucksensor Folgendes umfasst:
ein Substrat (130); und
eine Membran (120), die dem Substrat gegenüberliegt, wobei die Tiefe ein Abstand zwischen dem Substrat und der Membran ist.

3. System nach Anspruch 2, wobei der Drucksensor ferner Folgendes umfasst:
eine Abstandhalterschicht (530), die zwischen der Membran und dem Substrat angeordnet ist, wobei die Abstandhalterschicht, das Substrat und die Membran miteinander verbunden sind, um den optischen Hohlraum zu definieren; und
eine Blende (540), die an der Membran befestigt ist und eine Öffnung für die zumindest zwei nichtüberlagernden Strahlen aus der Vielzahl von Strahlen umfasst, damit diese auf den optischen Hohlraum auftreffen.

4. System nach Anspruch 1, wobei der Drucksensor Folgendes umfasst:
ein Substrat (130), das einen Abschnitt des optischen Hohlraums ausbildet;
einen Abstandhalter (530), der eine erste zentrale Öffnung umfasst, die einen zweiten Abschnitt des optischen Hohlraums ausbildet;
eine Membran (120), die an dem Abstandhalter befestigt ist und einen dritten Abschnitt des optischen Hohlraums ausbildet; und
eine Blende (540), die eine zweite zentrale Öffnung in Ausrichtung mit der ersten zentralen Öffnung umfasst.

5. System nach Anspruch 4, wobei die erste zentrale Öffnung kreisförmig ist und einen ersten Durchmesser von zwischen 0,19 mm und 0,35 mm aufweist und wobei die zweite Öffnung kreisförmig ist und einen zweiten Durchmesser von zwischen 0,19 mm und 0,35 mm aufweist, der kleiner oder gleich dem ersten Durchmesser ist.

6. System nach Anspruch 3, wobei die Blende Passermarken (920) umfasst.

7. System nach Anspruch 1, wobei der optische Hohlraum einen kreisförmigen Querschnitt aufweist.

8. System nach Anspruch 1, wobei der Prozessor ausgelegt ist, um Winkelfehlausrichtungen zwischen dem Spektrometer und dem Drucksensor basierend auf der Ausgabe zu korrigieren.

9. System nach Anspruch 1, wobei der Prozessor ferner ausgelegt ist, um einen lateralen Verschiebungsfehler zwischen dem Spektrometer und dem Drucksensor basierend auf der Ausgabe zu korrigieren.

10. Verfahren (1100) zum Messen eines Augeninnendrucks eines Auges (360) unter Verwendung eines Drucksensors (110), der einen optischen Hohlraum (140) aufweist und in das Auge implantiert ist, wobei das Verfahren Folgendes umfasst:
Ausstrahlen von Licht aus einer Lichtquelle (352) in das Auge, die ausgelegt ist, um eine Vielzahl von Strahlen (354) auszusenden, die ausgelegt sind, sodass zumindest zwei nichtüberlagernde Strahlen auf den Drucksensor auftreffen und von diesem reflektiert werden;
Empfangen von Licht, das von den zumindest zwei nichtüberlagernden Strahlen in ein Spektrometer (359) reflektiert wurde, um ein Spektrum zu erzeugen, wobei das Spektrum basierend auf einer Tiefe des optischen Hohlraums des Drucksensors variiert; und
Kommunizieren einer Ausgabe basierend auf dem Spektrum an einen Prozessor (380), der ausgelegt ist, um die Ausgabe zu empfangen und basierend auf der Ausgabe den Augeninnendruck des Auges zu schätzen.

11. Verfahren nach Anspruch 10, wobei der Drucksensor Folgendes umfasst:
ein Substrat (130);
eine Abstandhalterschicht (530), die an dem Substrat befestigt ist und eine erste zentrale Öffnung umfasst;
eine Abdeckung (520), die ein semi-reflektierendes Material umfasst, wobei die Abdeckung an der Abstandhalterschicht befestigt ist, und wobei ein Abschnitt der Abdeckung eine flexible Membran (120) bildet; und
eine Blende (540), die an der Abdeckung befestigt ist und eine zweite zentrale Öffnung umfasst, wobei die zweite zentrale Öffnung einen Umfang der flexiblen Membran ausbildet.

12. Verfahren nach Anspruch 10, wobei das Aussenden der Vielzahl von Strahlen aus dem Aussenden von räumlich kohärentem Licht in einer Strahlenanordnung besteht, wobei die Strahlen so bemessen und beabstandet sind, dass die zumindest zwei benachbarten Strahlen durch einen Abstand von weniger als eine Querschnittsabmessung des Drucksensors getrennt sind.

13. Verfahren nach Anspruch 12, wobei die Lichtquelle eine Bandbreite von etwa 30 nm aufweist, und der optische Hohlraum des Drucksensors eine Hohlraumtiefe von wenigen Mikrometern aufweist.

14. Verfahren nach Anspruch 12 oder Anspruch 13, wobei der Drucksensor Passermarken (920) umfasst, und wobei die Ausgabe auf Reflexionen eines oder mehrerer der Strahlen von den Passermarken basiert.

15. Verfahren nach Anspruch 12 oder Anspruch 13, wobei der Prozessor ferner ausgelegt ist, um eine Winkelfehlausrichtung zwischen dem Spektrometer und dem Drucksensor zu korrigieren.

## Revendications

1. Système de mesure de pression intraoculaire (300), comprenant :
un capteur de pression (110) pouvant être implanté dans un œil (360), dans lequel le capteur de pression définit une cavité optique (140) avec une profondeur qui varie en fonction d'une pression intraoculaire de l'œil ; et
un dispositif externe (150, 350) comprenant :
une source de lumière (352) configurée pour émettre une pluralité de faisceaux (354) de telle sorte qu'au moins deux faisceaux ne se chevauchant pas de la pluralité de faisceaux frappent et soient réfléchis à partir du capteur de pression ;
un spectromètre (359) configuré pour recevoir de la lumière réfléchie provenant des au moins deux faisceaux ne se chevauchant pas de la pluralité de faisceaux et produire un résultat sur la base de la lumière réfléchie provenant des au moins deux faisceaux ne se chevauchant pas de la pluralité de faisceaux, dans lequel le résultat varie sur la base de la profondeur de la cavité optique ; et
un processeur (380) configuré pour recevoir le résultat et, sur la base du résultat, estimer la pression intraoculaire de l'œil.

2. Système selon la revendication 1, dans lequel le capteur de pression comprend :
un substrat (130) ; et
une membrane (120) opposée au substrat, dans lequel la profondeur est une distance entre le substrat et la membrane.

3. Système selon la revendication 2, dans lequel le capteur de pression comprend en outre :
une couche d'espacement (530) située entre la membrane et le substrat, dans lequel la couche d'espacement, le substrat et la membrane sont liés pour définir la cavité optique ; et
une lunette (540) fixée à la membrane et comprenant une ouverture pour que les au moins deux faisceaux ne se chevauchant pas de la pluralité de faisceaux frappent la cavité optique.

4. Système selon la revendication 1, dans lequel le capteur de pression comprend :
un substrat (130) formant une partie de la cavité optique ;
un élément d'espacement (530) comprenant une première ouverture centrale formant une deuxième partie de la cavité optique ;
une membrane (120) fixée à l'élément d'espacement et formant une troisième partie de la cavité optique ; et
une lunette (540) comprenant une seconde ouverture centrale en alignement avec la première ouverture centrale.

5. Système selon la revendication 4, dans lequel la première ouverture centrale est circulaire et présente un premier diamètre compris entre 0,19 mm et 0,35 mm, et la seconde ouverture centrale est circulaire et présente un second diamètre compris entre 0,19 mm et 0,35 mm qui est égal ou inférieur au premier diamètre.

6. Système selon la revendication 3, dans lequel la lunette comprend des marqueurs de repère (920)

7. Système selon la revendication 1, dans lequel la cavité optique présente une section transversale circulaire.

8. Système selon la revendication 1, dans lequel le processeur est configuré pour corriger un désalignement angulaire entre le spectromètre et le capteur de pression sur la base du résultat.

9. Système selon la revendication 1, dans lequel le processeur est en outre configuré pour corriger une erreur de déplacement latéral entre le spectromètre et le capteur de pression sur la base du résultat.

10. Procédé (1100) pour mesurer une pression intraoculaire d'un œil (360) en utilisant un capteur de pression (110) présentant une cavité optique (140) et implanté dans l'œil, le procédé comprenant les étapes consistant à :
émettre de la lumière dans l'œil à partir d'une source de lumière (352) configurée pour émettre une pluralité de faisceaux (354) configurés de telle sorte qu'au moins deux faisceaux ne se chevauchant pas frappent et soient réfléchis à partir du capteur de pression ;
recevoir de la lumière réfléchie à partir des au moins deux faisceaux ne se chevauchant pas dans un spectromètre (359) pour produire un spectre, dans lequel le spectre varie sur la base d'une profondeur de la cavité optique du capteur de pression ; et
communiquer un résultat sur la base du spectre à un processeur (380) configuré pour recevoir le résultat et, sur la base du résultat, estimer la pression intraoculaire de l'œil.

11. Procédé selon la revendication 10, dans lequel le capteur de pression comprend :
un substrat (130) ;
une couche d'espacement (530) fixée au substrat et comprenant une première ouverture centrale ;
un couvercle (520) comprenant un matériau semi-réfléchissant, dans lequel le couvercle est fixé à la couche d'espacement, et dans lequel une partie du couvercle forme une membrane flexible (120) ; et
une lunette (540) fixée au couvercle et comprenant une seconde ouverture centrale, dans lequel la seconde ouverture centrale forme un périmètre de la membrane flexible.

12. Procédé selon la revendication 10,
dans lequel l'émission de la pluralité de faisceaux réside dans l'émission d'une lumière spatialement cohérente dans un réseau de faisceaux, dans lequel les faisceaux sont dimensionnés et espacés de telle sorte qu'au moins deux faisceaux adjacents sont séparés d'une distance inférieure à une dimension de section transversale du capteur de pression.

13. Procédé selon la revendication 12, dans lequel la source de lumière présente une largeur de bande d'environ 30 nm, et la cavité optique du capteur de pression présente une profondeur de cavité de quelques microns.

14. Procédé selon la revendication 12 ou la revendication 13, dans lequel le capteur de pression inclut des marqueurs de repère (920), et dans lequel le résultat est basé sur des réflexions d'un ou plusieurs des faisceaux à partir des marqueurs de repère.

15. Procédé selon la revendication 12 ou la revendication 13, dans lequel le processeur est en outre configuré pour corriger un désalignement angulaire entre le spectromètre et le capteur de pression.
